# EUROPEAN PATENT APPLICATION

(11) **EP 4 122 528 A1**
(43) Date of publication of application: **25.01.2023**
(21) Application number: 21187062.1
(22) Date of filing: 21.07.2021
(51) Int. Cl.: A61N 1/05

(54) **IMPLANTABLE ELECTRODE**

(71) Applicant: BIOTRONIK SE & Co. KG, 12359 Berlin (DE)
(72) Inventor: Dörr, Thomas, 12437 Berlin (DE)

(57) **Abstract**

The present invention relates to an implantable electrode comprising an elongated electrode lead (2) and a connecting plug (3) at a first terminus of the electrode lead (2), wherein the connecting plug (3) allows an electric connection of the electrode lead (2) to an implantable medical device (13), wherein the electrode lead (2) has a first cross-sectional maximum diameter (D1) and the connecting plug (3) has a second cross-sectional maximum diameter (D2). According to an aspect of the present invention, the second cross-sectional maximum diameter (D2) is equal to or smaller than the first cross-sectional maximum diameter (D1).

## Description

The present invention relates to an implantable electrode according to the preamble of claim 1 and to an implantable medical device comprising such an electrode according to claim 10.

Implantable electrodes known from prior art are often implanted via a tunneling technique. Since the connecting plug of these electrodes is bigger than an electrode lead of the electrodes, it would be necessary to provide a sufficiently big tunneling catheter in order to also guide the connecting plug through the tunneling catheter. However, it is desirable to use tunneling catheters as small as possible for implantation purposes in order to reduce the risk of injuries within the patient to whom the implantable electrode is to be implanted.

Consequently, prior art implantable electrodes can only be moved in a single direction through a tunneling catheter, namely with their electrode tip facing forward. As a result, the implantation procedure is partially limited and is often more complex than necessary from a purely medical point of view. Furthermore, it is necessary to slit, split or peel the tunneling catheter to remove it from the implanted electrode that has been moved inside the tunneling catheter. Such slitting, splitting or peeling is connected to the risk of an electrode dislocation. It additionally circumvents the reuse of the tunneling catheter.

It is an object of the present invention to facilitate the implantation of an implantable electrode, in particular to enable the possibility of implanting an electrode including all its components in any desired direction.

This object is achieved with an implantable electrode having the features explained in the following. Such an implantable electrode comprises an elongated electrode lead and a connecting plug at a first terminus of the electrode lead. This first terminus is opposite to a second terminus of the electrode lead that typically constitutes the electrode tip. In many instances, one or more electrode poles are arranged within the region of the second terminus of the electrode lead. The connecting plug allows an electric connection of the electrode lead to an implantable medical device. For this purpose, the connecting plug is inserted into a corresponding connecting socket of an implantable medical device. The electrode lead has a first cross-sectional maximum diameter. Likewise, the connecting plug has a second cross-sectional maximum diameter.

According to an aspect of the present invention, the second cross-sectional maximum diameter is equal to or smaller than the first cross-sectional maximum diameter. By such an arrangement, it will always be possible to move the connecting plug of the implantable electrode through a tunneling catheter whenever the elongated electrode lead was or could have been guided through the same tunneling catheter. Thus, by designing the connecting plug of the implantable electrode smaller than in case of prior art electrodes, a much higher flexibility of the applied implantation technique is made possible. To be more precise, it is possible to implant the electrode according to the present invention either with its connecting plug facing forward or with its electrode tip facing forward. No specific tunneling catheter is necessary for this purpose. Rather, tunneling catheters known from prior art can also be used for the presently claimed implantable electrode since the overall dimensions of the electrode are not changed. Rather, only the relative dimensions of the connecting plug have been reduced with respect to the relative dimensions of the electrode lead.

Since this electrode enables an implantation without slitting, splitting or peeling the tunneling catheter after having moved the electrode to its intended site of implantation, the risk of an electrode dislocation due to such slitting, splitting or peeling is completely avoided. The overall risk of an electrode dislocation is - taking into account the small risk of such dislocation due to a relative movement between a tunneling catheter and the electrode after having positioned the electrode at its intended site of implantation - significantly reduced with respect to prior art techniques.

Thus, the presently claimed implantable electrode increases the possibilities of tunneling an electrode for the purpose of implantation and significantly facilitates the necessary implantation procedure.

In an embodiment, the electrode lead has a substantially constant cross-sectional diameter over its entire length. Thus, the electrode lead does not comprise any bulbs or protrusions that would impair its implantation. Then, the first cross-sectional maximum diameter (i.e., the cross-sectional maximum diameter of the electrode lead) corresponds to the general or overall cross-sectional diameter of the electrode lead.

In an embodiment, the connecting plug has a substantially constant cross-sectional diameter over its entire length. Thus, the connecting plug does not comprise protrusions or bulbs in this embodiment. Then, the second cross-sectional maximum diameter (i.e., the cross-sectional maximum diameter of the connecting plug) corresponds to the overall cross-sectional diameter of the connecting plug.

In an embodiment, the connecting plug has an opening through which a towing element, such as a towing rope, can be guided. Then, it is possible to connect such a towing element to the connecting plug and to pull the electrode with the help of the towing element through a tunneling catheter to its intended site of implantation. Prior art electrodes do not comprise such an opening for the connection of a towing element since they were never intended to be guided with their connecting plug facing forward through a tunneling catheter during implantation. Rather, they were always implanted with their electrode tip facing forward.

In an embodiment, the opening is provided in a connecting pin of the connecting plug that is arranged at a free end of the connecting plug. This free end of the connecting plug typically constitutes the extreme end of the electrode in the region of the connecting plug (like the electrode tip constitutes another extreme end at the opposite side of the electrode along its longitudinal direction of extension). With respect to the connecting plug, the free end of the connecting plug is opposite a transition area between the connecting plug and the electrode lead. This transition area represents a second end of the electrode plug, which is, however, not a free end of the connecting plug, but an end connected to the electrode lead of the electrode. An arrangement of the opening at the extreme end of the connecting plug (and therewith also at one of the extreme ends of the electrode) facilitates the guiding of the whole electrode with a towing element that has been connected to the electrode by guiding it through the opening and securing it to a part of the connecting plug surrounding the opening.

In an embodiment, the connecting pin has a third cross-sectional maximum diameter being equal to or smaller than the second cross-sectional maximum diameter. Thus, the provision of the connecting pin does not impart the general possibility of guiding the electrode through a tunneling catheter. Rather, the maximum cross-sectional diameter of the electrode lead is still the limiting and determining factor for a required inner diameter of such tunneling catheter.

In an embodiment, the connecting plug is made sufficiently flexible to allow a bending so that the connecting plug substantially does not impart a movement of the electrode along a curved trajectory. This facilitates the implantation of the electrode. For this purpose, the connecting plug has a length and a stiffness allowing a bending of the connecting plug about a bending radius of at least 15 cm, in particular of at least 20 cm, in particular of at least 25 cm. In an embodiment, the length and stiffness of the connecting plug allow a bending of the connecting plug about a bending radius lying in a range of from 15 cm to 50 cm, in particular of from 20 cm to 45 cm, in particular of from 25 cm to 40 cm, in particular of from 30 cm to 35 cm. If the connecting plug is too short and too rigid, a bending about such a bending radius will not be possible anymore. Since the length and the stiffness of the connecting plug depend on each other in order to achieve a bending possibility as defined above, no concrete values of length and stiffness can be indicated.

In an embodiment, the connecting plug comprises at least two contact poles and at least one intermediate section arranged between the at least two contact poles or arranged adjacent to one of the at least two contact poles. The intermediate section comprises a flexible plastic material, such as silicon or polyurethane (PU), in this embodiment. Such an arrangement increases the flexibility of the connecting plug and facilitates an achievement of the above-defined bending properties. The contact poles are typically rigid, i.e. non-flexible. Then, the flexibility of the connecting plug is given by the at least one intermediate section that allows - if arranged between two contact poles - a relative movement of the contact poles with respect to each other.

In an embodiment, the implantable electrode is a sensor lead for determining at least one physiologic parameter of a human or animal patient. In another embodiment, the implantable electrode is an antenna, such as an antenna of an implantable medical device. In another embodiment, the implantable electrode is an electrode appropriate for an electric therapy of a human or animal heart, for a cardioversion/defibrillation of a human or animal heart, for an antibradycardic cardiac stimulation of a human or animal heart, and/or for an antitachycardic stimulation of a human or animal heart. In a further embodiment, the implantable electrode is an electrode of a non-transvenously implantable defibrillation arrangement, for a substernally implantable defibrillation arrangement, for a subcutaneously implantable defibrillation arrangement, for an intramuscularly implantable defibrillation arrangement, and/or for a submuscularly implantable defibrillation arrangement. These intended uses of the implantable electrode take particular advantage of an easier implantation method as enabled by the presently claimed implantable electrode.

In an aspect, the present invention relates to an implantable medical device comprising an implantable electrode according to the preceding explanations. The implantation of such a medical device is particularly facilitated by an implantable electrode according to an aspect of the present invention since it offers a higher flexibility during the implantation and reduces the risk of physiologic undesired electrode dislocations upon the electrode implantation.

In an embodiment, the implantable medical device is a sensor device for determining at least one physiologic parameter of a human or animal patient. In another embodiment, the implantable medical device is a device for stimulating a human or animal heart. In an embodiment, the implantable medical device is a non-transvenously implantable defibrillation device. In an embodiment, the implantable medical device is a substernally implantable defibrillation device. In an embodiment, the implantable medical device is a subcutaneously implantable defibrillation device. A particular appropriate subcutaneously implantable defibrillation device is a subcutaneously implantable cardioverter/defibrillator (S-ICD). In an embodiment, the implantable medical device is an intramuscularly implantable defibrillation device. In an embodiment, the implantable medical device is a submuscularly implantable defibrillation device.

In an aspect, the present invention relates to a method for implanting an implantable electrode according to the preceding explanations into a body of a human or animal patient in need of such implantation. The method comprises the steps explained in the following.

In a first step, a skin incision is made into the patient's skin.

Afterwards, a tunneling catheter is moved through the skin incision under the patient's skin.

Before, concomitantly with our after moving the tunneling catheter, the implantable electrode is moved within the tunneling catheter towards its intended position of implantation. The implantable electrode comprises - as outlined above - an elongated electrode lead and a connecting plug at a first terminus of the electrode lead. The connecting plug allows an electric connection of the electrode lead to an implantable medical device.

Afterwards, the tunneling catheter is removed from around the implantable electrode. For this purpose, the tunneling catheter is withdrawn over the connecting plug of the implantable electrode without mechanically rupturing or otherwise impairing the tunneling catheter. Expressed in other words, no slitting, splitting or peeling of the tunneling catheter takes place prior to or during this removing step.

Afterwards, the connecting plug is connected to an implantable medical device. Finally, the skin incision is closed, e.g., by suturing, clamping, or by applying an appropriate adhesive tape above it.

In contrast to prior art techniques, the tunneling catheter can be simply moved over the connecting plug of the implantable electrode since the connecting plug has a maximum cross-sectional diameter that is not bigger than the maximum cross-sectional diameter of the electrode lead of the implantable electrode. Since the tunneling catheter remains intact after having removed it from the implanted electrode, it can be reused, in particular during the same operation for implanting another electrode into the same patient. This reduces the required material and thus the associated costs.

In an embodiment, the implantable electrode is moved within the tunneling catheter with its connecting plug facing forward. To give an example, the electrode poles of the electrode can be implanted in the area of the sternum of the patient and the connecting plug can then be moved forward to a subcutaneously implanted medical device to be finally connected to this device. Such an implantation makes use of an opposite implantation direction with respect to prior art techniques. It facilitates the correct placement of the electrode poles at the intended site of implantation. In addition, a connection between the connecting plug and the associated medical device is only established after having correctly placed the electrode within the patient's body. According to prior art techniques, a connection between such a connecting plug of an electrode is established prior to positioning the electrode in its final implantation position.

In an embodiment, the patient additionally has a thorax comprising a heart and a sternum, wherein the thorax is located in a thoracic region of the patient. In this embodiment, the implantable medical device is configured to sense electric signals from the patient's heart and optionally to stimulate the patient's heart with electric signals. The implantable electrode is implanted, in this embodiment, in the thoracic region of the patient above the patient's sternum. Such a site of implantation is particularly appropriate to sense electric cardiac signals and to stimulate the patient's heart from outside the heart, i.e., without accessing an interior of the heart. Such site of implantation is particularly appropriate for a subcutaneously implanted cardioverter/defibrillator (S-ICD).

In an embodiment, the implantable electrode is implanted in a subcutaneous, an intramuscular, or a submuscular manner. When placing the electrode with its electrode poles above the patient's sternum, the electrode is typically implanted in a subcutaneous manner.

All embodiments of the implantable electrode can be combined in any desired manner and can be transferred either individually or in any desired combination to the implantable device and to the implantation method. Likewise, all embodiments of the implantable medical device can be combined in any desired manner and can be transferred either individually or in any desired combination to the implantable electrode and to the implantation method. Finally, all embodiments of the implantation method can be combined in any desired manner and can be transferred either individually or in any arbitrary combination to the implantable electrode or to the implantable medical device.

Further details of aspects of the present invention will be explained in the following with respect to exemplary embodiments and accompanying Figures. In the Figures:
- Fig. 1A: shows an embodiment of an implantable electrode;
- Fig. 1B: shows a maximum cross-sectional diameter of an electrode lead of the electrode of Fig. 1A;
- Fig. 1C: shows a maximum cross-sectional diameter of a connecting plug of the implantable electrode of Fig. 1A;
- Fig. 2A: shows a detail of a proximal portion of an implantable electrode;
- Fig. 2B: shows a maximum cross-sectional diameter of a connecting pin of the implantable electrode of Fig. 2A;
- Fig. 3: shows an implantable medical device with a connected electrode; and
- Fig. 4: schematically shows the torso of a patient, illustrating an exemplary embodiment of an implantation site of an implantable electrode as well as the incision utilized for implanting the implantable electrode.

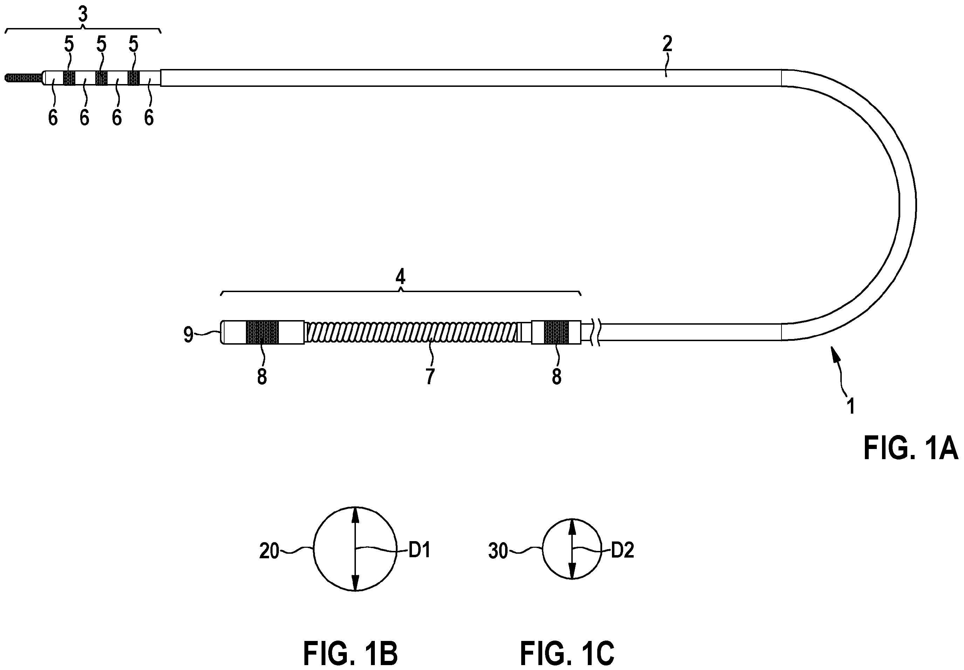

Fig. 1A shows an implantable electrode 1 comprising an elongated electrode lead 2 and a connecting plug 3 arranged in a proximal end portion of the implantable electrode 1 as well as an electrode pole region 4 in a distal end region of the implantable electrode 1. The electrode lead 2 comprises a conductive part and an insulating part, which are not separately illustrated in the depiction of Fig. 1A.

The connecting plug 3 comprises a plurality of contact poles 5 and a plurality of intermediate sections 6 arranged between these contact poles 5 or adjacent to at least one of the contact poles 5. The implantable electrode 1 is intended to be used as defibrillation electrode. For this purpose, its electrode pole region 4 comprises a shock coil 7 representing a first electrode pole as well as two sensing electrodes 8 being arranged adjacent to the shock coil 7 at each side thereof. The shock coil 7 and the sensing electrodes 8 are each connected with one of the contact poles 5 by separate leads forming part of the electrode lead 2.

Fig. 1B schematically shows a cross section 20 of the electrode lead 2 shown in Fig. 1A. This cross section 20 has a maximum diameter D1.

Fig. 1C schematically shows a cross section 30 of the connecting plug 3 of the implantable electrode 1 of Fig. 1A. This cross section 30 of the connecting plug 3 has a maximum diameter D2. By comparing Figures 1B and 1C, it is apparent that the maximum diameter D2 of the cross section 30 of the connecting plug 3 is smaller than the maximum diameter D1 of the cross section 20 of the electrode lead 2. Due to this smaller cross section D2, it is possible to move the implantable electrode 1 through a tunneling catheter without the connecting plug 3 being an obstacle or limiting condition for an inner diameter of such tunneling catheter.

It is possible to implant the implantable electrode 1 through a tunneling catheter either with its electrode tip 9 facing forward or with the connecting plug 3 facing forward. Furthermore, after having placed the implantable electrode 1 at its intended site of implantation, an according tunneling catheter can be removed from the implantable electrode 1 without the need of slitting, splitting or peeling such tunneling catheter. Rather, it can be simply withdrawn over the connecting plug 3.

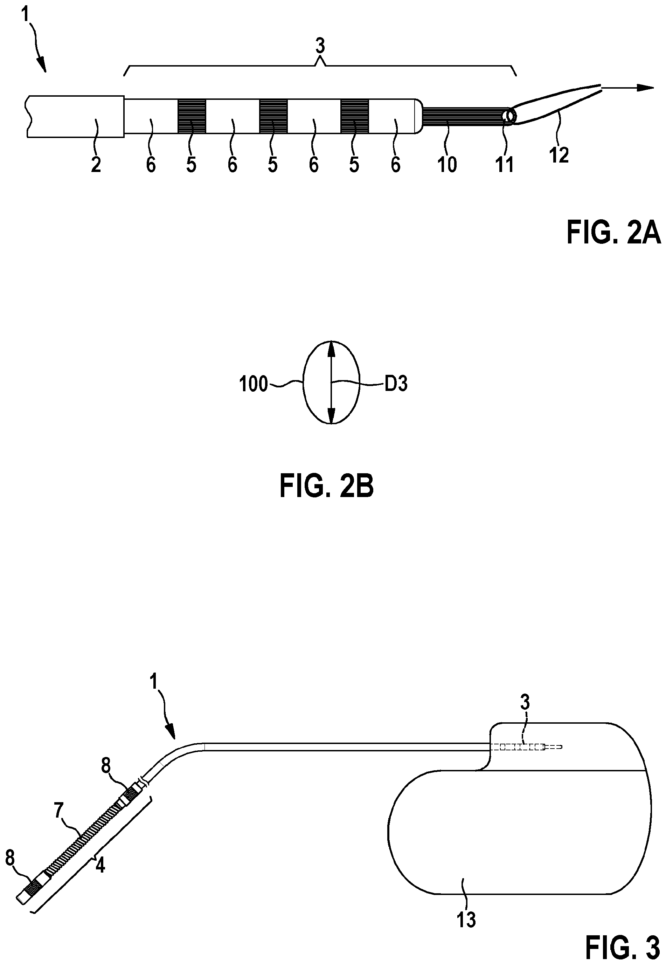

Fig. 2A shows the proximal end portion of another embodiment of an implantable electrode 1. In this and in all following Figures, similar elements will be denoted with the same numeral references as in the preceding Figures.

The proximal end portion of the implantable electrode 1 comprises an insulated electrode lead 2 as well as a connecting plug 3. The connecting plug 3 comprises three contact poles 5 as well as four intermediate sections 6 lying in between these contact poles 5 or directly adjacent to at least one of the contact poles 5. The connecting plug 3 further comprises a connecting pin 10 having an opening 11 at an extreme end thereof. The opening 11 serves for guiding a towing rope 12 through it. This towing rope 12 makes it possible to pull the whole implantable electrode 1 during the implantation procedure in the direction indicated with an arrow in Fig. 2A. This facilitates an implantation of the electrode 1 with its connecting plug 3 facing forward.

Fig. 2B shows a cross section 100 of the connecting pin 10. This cross section 100 has a maximum diameter D3. When comparing Fig. 2B with Figures 1B and 1C, it is apparent that the maximum diameter D3 of the cross section 100 of the connecting pin 10 is smaller than the maximum diameter D1 and the maximum diameter D2. Thus, the provision of the connecting pin 10 does not impart the easy implantation of the implantable electrode 1.

Fig. 3 shows a subcutaneously implantable cardioverter-defibrillator S-ICD 13 as example of an implantable medical device. It comprises an implantable electrode 1, e.g., the implantable electrode 1 of Fig. 1A. The connecting plug 3 of the implantable electrode 1 is inserted into a socket of the S-ICD 13. And electrode pole region 4 comprising a shock coil 7 and two adjacent sensing electrodes 8 is, in its implanted state, placed near to the heart to be stimulated by the S-ICD 13.

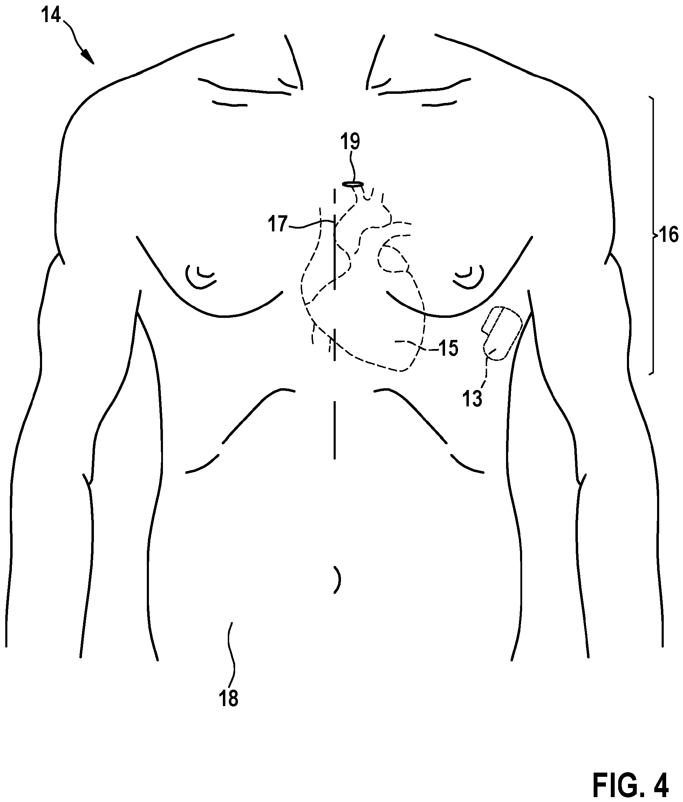

Fig. 4 shows a torso of a patient 14. The patient's heart 15 is located in a thoracic region 16 below a sternum 17 of the patient 14. A skin 18 covers the heart 15 and the sternum 17. Through a skin incision 19 in the region of the sternum 17, an implantable electrode (for illustrative purposes not shown in Fig. 4, but depicted in Figures 1A and 2A to which reference is made for the following explanations) can be implanted in the thoracic region 16 of the patient 14. For this purpose, the implantable electrode is guided through an appropriate implantation tool such as a tunneling catheter with its connecting plug 3 facing forward through the skin incision 19 in a subcutaneous manner to an S-ICD 13.

After having placed the electrode pole region 4 at its intended site of implantation (i.e., above the sternum 17 of the patient 14), the connecting plug 3 of the implantable electrode 1 is connected with the S-ICD 13. Thus, in contrast to prior art techniques, it is not necessary to establish first a connection between the implantable electrode 1 and the S-ICD 13 and afterwards to correctly position the implantable electrode above the patient's heart 15. Rather, the implantation steps are directly opposite. This significantly facilitates a correct and reliable implantation of the implantable electrode 1 and avoids an undesired dislocation of the implantable electrode 1 during the implantation. Furthermore, the tunneling catheter can be simply removed by withdrawing it over the connecting plug 3 of the implantable electrode 1 without having the need of slitting, splitting or peeling it so that another risk of dislocating the electrode 1 is also avoided.

After having completed the implantation of the implantable electrode 1, the skin incision 19 is closed.

## Claims

1. Implantable electrode (1) comprising an elongated electrode lead (2) and a connecting plug (3) at a first terminus of the electrode lead (2), wherein the connecting plug (3) allows an electric connection of the electrode lead (2) to an implantable medical device (13), wherein the electrode lead (2) has a first cross-sectional maximum diameter (D1) and the connecting plug (3) has a second cross-sectional maximum diameter (D2),
**characterized**
**in that** the second cross-sectional maximum diameter (D2) is equal to or smaller than the first cross-sectional maximum diameter (D1).

2. Implantable electrode according to claim 1, **characterized in that** the electrode lead (3) has a substantially constant cross-sectional diameter (D1) over its entire length.

3. Implantable electrode according to claim 1 or 2, **characterized in that** the connecting plug (3) has a substantially constant cross-sectional diameter (D2) over its entire length.

4. Implantable electrode according to any of the preceding claims, **characterized in that** the connecting plug (3) has an opening (11) through which a towing element (12) can be guided.

5. Implantable electrode according to claim 4, **characterized in that** the opening (11) is provided in a connecting pin (10) of the connecting plug (3) that is arranged at a free end of the connecting plug (3) opposite a transition area between the connecting plug (3) and the electrode lead (2).

6. Implantable electrode according to claim 5, **characterized in that** the connecting pin (10) has a third cross-sectional maximum diameter (D3) being equal to or smaller than the second cross-sectional maximum diameter (D2).

7. Implantable electrode according to any of the preceding claims, **characterized in that** the connecting plug (3) has a length and a stiffness allowing a bending of the connecting plug (3) about a bending radius of at least 15 cm.

8. Implantable electrode according to any of the preceding claims, **characterized in that** the connecting plug (3) comprises at least two contact poles (5) and at least one intermediate section (6) arranged between the at least two contact poles (5) or directly adjacent to one of the at least two contact poles (5), wherein the intermediate section (6) comprises a flexible plastic material.

9. Implantable electrode according to any of the preceding claims, **characterized in that** the implantable electrode (1) is a sensor lead for determining at least one physiologic parameter of a human or animal patient, an antenna or an electrode for at least one of an electric therapy of a human or animal heart, a cardioversion/defibrillation of a human or animal heart, an antibradycardic stimulation of a human or animal heart, an antitachycardic stimulation of a human or animal heart, a non-transvenously implantable defibrillation arrangement, a substernally implantable defibrillation arrangement, a subcutaneously implantable defibrillation arrangement, an intramuscularly implantable defibrillation arrangement, and a submuscularly implantable defibrillation arrangement.

10. Implantable medical device comprising an implantable electrode (1) according to any of the preceding claims.

11. Implantable medical device according to claim 10, **characterized in that** the implantable medical device (13) is a sensor device for determining at least one physiologic parameter of a human or animal patient, a device for stimulating a human or animal heart, a non-transvenously implantable defibrillation device, a substernally implantable defibrillation device, a subcutaneously implantable defibrillation device, an intramuscularly implantable defibrillation device, or a submuscularly implantable defibrillation device.

12. A method for implanting an implantable electrode according to any of claims 1 to 9 into a body of a human or animal patient (14) in need of such implantation, wherein the patient (14) has a skin (18), wherein the method comprises the following steps:
a) performing a skin incision (19) into the patient's skin (18);
b) moving a tunneling catheter through the skin incision (19) under the patient's skin (18);
c) moving the implantable electrode (1) within the tunneling catheter towards the intended position of implantation, wherein the implantable electrode (1) comprises an elongated electrode lead (2) and a connecting plug (3) at a first terminus of the electrode lead (2), wherein the connecting plug (3) allows an electric connection of the electrode lead to an implantable medical device (13);
d) removing the tunneling catheter from around the implantable electrode (1) by withdrawing it over the connecting plug (3) of the implantable electrode (1) without mechanically impairing the tunneling catheter;
e) connecting the connecting plug (3) to an implantable medical device (13); and
f) closing the skin incision (19).

13. The method according to claim 12, **characterized in that** the implantable electrode (1) is moved within the tunneling catheter with the connecting plug (3) facing forward.

14. The method according to claim 12 or 13, **characterized in that** the patient (14) additionally has a thorax comprising a heart (15) and a sternum (17), wherein the thorax is located in a thoracic region (16) of the patient (14), wherein the implantable medical device (1) is configured to sense electrical signals from the patient's heart (15) and optionally to stimulate the patient's heart (15) with electrical signals, wherein the implantable electrode (1) is implanted in the thoracic region (16) of the patient (14) above the patient's sternum (17).

15. The method according to any of claims 12 to 14, **characterized in that** the implantable electrode (1) is implanted in a subcutaneous, an intramuscular, or a submuscular manner.
